# EUROPÄISCHE PATENTSCHRIFT

(11) **EP 1 198 457 B1**
(45) Veröffentlichungstag und Bekanntmachung des Hinweises auf die Patenterteilung: **14.12.2005**
(21) Anmeldenummer: 00958303.0
(22) Anmeldetag: 27.07.2000
(51) Int. Cl.: C07D 221/06, A61K 7/13

(54) **QUATERNIERTE AZAFLUORENONE UND DEREN VERWENDUNG IN MITTELN ZUM FÄRBEN VON KERATINHALTIGEN FASERN**
QUATERNIZED AZAFLUORENONES AND THEIR USE IN AGENTS FOR DYING FIBERS CONTAINING KERATIN
AZAFLUORENONES QUATERNARISES ET LEUR UTILISATION DANS DES AGENTS POUR COLORER DES FIBRES A BASE DE KERATINE

(30) Priorität: 06.08.1999 DE 19937301
(43) Veröffentlichungstag der Anmeldung: 24.04.2002
(73) Patentinhaber: Henkel Kommanditgesellschaft auf Aktien, 40589 Düsseldorf (DE)
(72) Erfinder: MÖLLER, Hinrich, 40789 Monheim (DE); OBERKOBUSCH, Doris, 40591 Düsseldorf (DE); HÖFFKES, Horst, 40595 Düsseldorf (DE)
(86) Internationale Anmeldenummer: PCT/EP2000/007226
(87) Internationale Veröffentlichungsnummer: WO 2001/010840

(56) Entgegenhaltungen:
- EP-A- 0 758 547
- WO-A-94/09002
- CH-A- 616 441
- DE-A- 2 051 318
- FR-A- 1 430 089
- FR-A- 2 073 388
- DATABASE CHEMABS [Online] CHEMICAL ABSTRACTS SERVICE, COLUMBUS, OHIO, US; PROSTAKOV, N. S. ET AL: "Reduction of quaternary salts of 3-azafluorene and 3-azafluorenone and their conversions into indenoindolizines" retrieved from STN Database accession no. 98:215441 XP002153246 & KHIM. GETEROTSIKL. SOEDIN. (1983), (2), 252-5 ,
- DATABASE CHEMABS [Online] CHEMICAL ABSTRACTS SERVICE, COLUMBUS, OHIO, US; MARDENBOROUGH, LEROY G. ET AL: "Identification of a novel antifungal agent based on a pharmacophoric group in cryptolepine" retrieved from STN Database accession no. 131:44995 XP002153511 & MED. CHEM. RES. (1999), 9(2), 118-132 ,
- DATABASE CHEMABS [Online] CHEMICAL ABSTRACTS SERVICE, COLUMBUS, OHIO, US; ZANDERSONS, A. ET AL: "Synthesis of substituted 5-oxoindeno[1,2-b]pyridinium salts" retrieved from STN Database accession no. 105:208733 XP002153512 & KHIM. GETEROTSIKL. SOEDIN. (1986), (1), 88-90 ,
- DATABASE CHEMABS [Online] CHEMICAL ABSTRACTS SERVICE, COLUMBUS, OHIO, US; KLOC, KRYSTIAN ET AL: "Reactions at the nitrogen atoms in azafluorene systems" retrieved from STN Database accession no. 91:123651 XP002153513 & CAN. J. CHEM. (1979), 57(12), 1506-10 ,
- DATABASE CHEMABS [Online] CHEMICAL ABSTRACTS SERVICE, COLUMBUS, OHIO, US; PROSTAKOV, N. S. ET AL: "2,5-Dimethyl-4-phenylpyridine in synthesis of substituted indolizines and indeno[2,1-f]indolizines" retrieved from STN Database accession no. 77:164401 XP002153514 & KHIM. GETEROTSIKL. SOEDIN. (1972), (9), 1220-3 ,
- DATABASE CHEMABS [Online] CHEMICAL ABSTRACTS SERVICE, COLUMBUS, OHIO, US; ABRAMENKO, P. I.: "Polymethine dyes derived from 2-azafluorene and their spectral properties" retrieved from STN Database accession no. 99:141527 XP002153247 & ZH. VSES. KHIM. O-VA. (1983), 28(3), 349-51 ,

## Beschreibung

Die Erfindung betrifft ein Mittel zum Färben von keratinhaltigen Fasern, insbesondere menschlichen Haaren, das quarternierte Azafluorenone enthält, die Verwendung dieser quartemierten Azafluorenone als färbende Komponente in Haarfärbemitteln sowie ein Verfahren zum Färben von keratinhaltigen Fasern, insbesondere menschlichen Haaren.

Für das Färben von keratinhakigen Fasern, z. B. Haaren, Wolle oder Pelzen, kommen im allgemeinen entweder direktziehende Farbstoffe oder Oxidationsfarbstoffe, die durchoxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrirnidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethy)amino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen quarternierten Azafluorenone zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Die Druckschrift WO-A1-94/09002 betrifft ein spezielles Carbapenem-substituiertes Azafluoren-9-on-Derivat mit antibakterieller Wirkung.

In der Veröffentlichung Med. Chem. Res. 1999, 9(2), 118-132 werden Cryptolepin-analoge Verbindungen zur Ermittlung von potenten, antibakteriellwirkenden Verbindungen beschrieben. 4-Methyl-4-azafluorenoniumiodid wird in diesem Kontext offenbart.

In der Veröffentlichung Khim. Geterotsikl. Soedin., 1983, 2, 252-255 wird die Synthese von 3-Azafluorenon-Salzen beschrieben.

In der Veröffentlichung Khim. Geterotsikl. Soedin., 1986, 1, 88-90 wird die Synthese von 5-Oxo-indeno[1,2-b]pyridinium-Salzen beschrieben.

In der Veröffentlichung Khim. Geterotsikl. Soedin., 1972, 9, 1220-1223 wird die Synthese von Indeno[2,1-f]indolizinen beschrieben.

Die Veröffentlichung Can. J. Chem., 1979, 57(12), 1506-1510 hat die Reaktivität von Azafluorenonen am Aza-Stickstoffatom zum Gegenstand.

Keine dieser Veröffentlichungen offenbart die Verwendung der erfindungsgemäßen quaternierten Azafluorenone in Mitteln zur Färbung keratinhaltiger Fasern.

Überraschenderweise wurde nun gefunden, daß die in der Formel I dargestellter, quartemierten Azafluorenone sich in Kombination mit mindestens einer weiteren Komponente, ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder CH-aktiven Verbindungen auch in Abwesenheit von oxidierenden Agentien hervorragend zum Färben von keratinhaltigen Fasern eignen. Der Einsatz von oxidierenden Agentien soll dabei jedoch nicht prinzipiell ausgeschlossen werden.

Ein Gegenstand der Erfindung ist ein Mittel zum Färben vonkeratinhaltigen Fasern, insbesondere menschlichen Haaren, enthaltend die quarternierten Azafluorenone mit der Formel I, 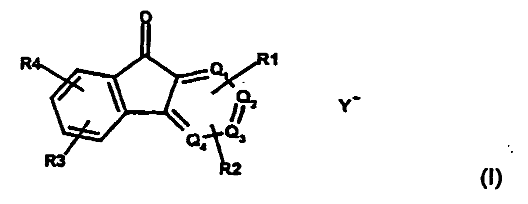 in der R¹, R², R³ und R⁴ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe, wobei auch zwei der Reste gemeinsam einen ankondensierten aromatischen Ring bilden können, Q₁, Q₂, Q₃ und Q₄ in der Summe drei Kohlenstoffatome und ein quartäres Stickstoffatom, das den Rest R⁵ mit der Bedeutung einer C₁₋₄-Alkyl-, C₁₋₄-Hydroxyalkyl-, C₁₋₄-Carboxyalkyl-, C₁₋₄-Sulfoalkyl-, einer Aryl-, Aralkyl- oder Heteroarylgruppe oder auch eines N-Oxids trägt, und
Y⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat, Tetrachlorzinkat oder Oxid eines N-Oxids bedeuten
in Kombination mit mindestens einer weiteren Komponente, ausgewählt aus Verbindungen mit primäres oder sekundärer Aminogruppe oder Hydroxygruppe, ausgewählt aus primären oder sekundären aromatischen Aminen, stickstoff haltigen heterocyclischen Verbindungen und aromatischen Hydroxy verbindungen, und/oder CH-aktiven Verbindungen.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Vorzugsweise sind die Verbindungen mit der Formel I ausgewählt aus der Gruppe der Trifluormethansulfonate, Benzolsulfonate, p-Toluolsulfonate, Methansulfonate, Perchlorate, Sulfate, Chloride, Bromide, Iodide, Tetrachlorzinkate, Hexafluorphosphate Tetrafluorborate von 1-Methyl-5-oxo-indeno[1,2-b]pyridinium, 4-methyl-4-azonio-9-fluoreno, 1-Ethyl-5-oxo-indeno[1,2-b]pyridinium, 1-Benzyl-5-oxo-indeno[1,2-b]pyridinium, 2-Methyl-5-oxo-indeno[1,2-c]pyridinium, 2-Methyl-9-oxo-indeno[2,1-c]pyridinium, 1-Methyl-9-oxo-ideno[2,1-b]pyridinium sowie deren beliebigen Gemischen.

Diese Substanzen sind zum großen Teil literaturbekannt oder im Handel erhältlich oder nach bekannten Syntheseverfahren, wie sie z. B. im Organikum, VEB deutscher Verlag der Wissenschaften, Berliln 1981, S. 260 beschrieben sind, herstellbar.

Die voranstehend genannten quartemierten Azafluorenone mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbungen mit erhöhter Brillanz und verbesserten Echtheitseigenschaften (Lichtechtheit, Waschechtheit, Reibechtheit) über einen weiten Nuancenbereich von gelb über gelbbraun, orange, braunorange, mittelbraun, dunkelbraun, violett, dunkelviolett bis hin zu dunkelgrünbraun und schwarz werden erzielt, wenn erfindungsgemäß die eingesetzten Verbindungen mit der Formel I 'gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder CH-aktiven Verbindungen, verwendet werden. Dies sind einerseits Verbindungen, die für sich alleine keratinhaltige Fasern nur schwach färben und erst gemeinsam mit den quartemierten Azafluorenonen der Formel I brillante Färbungen ergeben. Andererseits sind darunter aber auch Verbindungen, die bereits als Oxidationsfarbstoffvorprodukte eingesetzt werden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene quartemierte Azafluorenone der Formel I gemeinsam zum Einsatz kommen; ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von quartemierten Azafluorenonen der Formel I mit den genannten Verbindungen der Komponente B darstellen, als direktziehende Färbemittel. Derartige Reaktionsprodukte können z. B. durch kurzes Erwärmen der beiden Komponenten in stöchiometrischen Mengen in wäßrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Geeignete Verbindungen mit primärer oder sekundärer Aminogruppe als Komponente B sind z. B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, - phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 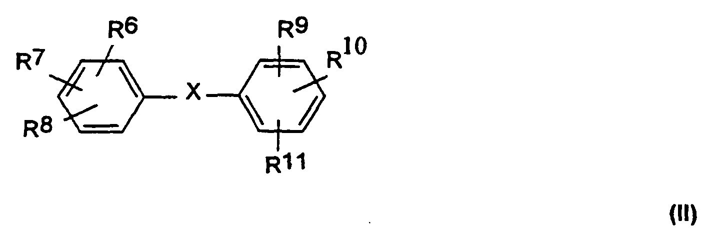 in der R⁶ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht, R⁷, R⁸, R⁹, R¹⁰ und R¹¹ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
X für eine direkte Bindung, eine gesättigte oder ungesättigte, ggf. durchHydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

**Z-(CH**_{**2**}**-Q-CH**_{**2**}**-Z')**_{**o**} **(III)** (III)

in der Q eine direkte Bindung, eine CH₂- oder CHOH-Gruppe bedeutet, Z und Z' unabhängig voneinander für ein Sauerstoffatom, eine NR¹²-Gruppe, worin R¹² Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und
o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorind, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie deren mit vorzugsweise anorganischen Säuren gebildeten physiologisch verträglichen Salze.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe derNitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel inwasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, lmidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung von quarternierten Azafluorenonen mit der Formel I, 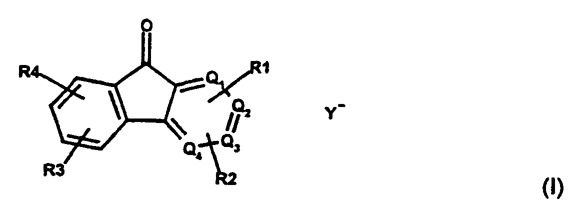 in der R¹, R², R³ und R⁴ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe, wobei auch zwei der Reste gemeinsam einen ankondensierten aromatischen Ring bilden können, Q₁, Q₂, Q₃ und Q₄ in der Summe drei Kohlenstoffatome und ein quartäres Stickstoffatom, das den Rest R⁵ mit der Bedeutung einer C₁₋₄-Alkyl-, C₁₋₄-Hydroxyalkyl-, C₁₋₄-Carboxyalkyl-, C₁₋₄-Sulfoalkyl-, einer Aryl-, Aralkyl- oder Heteroarylgruppe oder auch eines N-Oxids trägt, und
Y⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat, Tetrachlorzinkat oder Oxid eines N-Oxids bedeuten,
als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend,
A) mindestens ein quartemiertes Azafluorenon mit der Formel I, 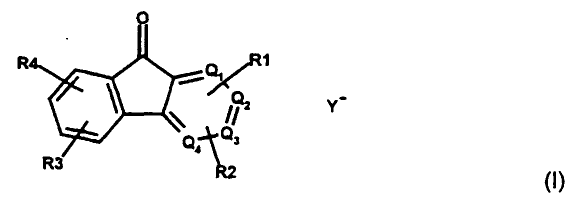 in der R¹, R², R³ und R⁴ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe, wobei auch zwei der Reste gemeinsam einen ankondensierten aromatischen Ring bilden können, Q₁, Q₂, Q₃ und Q₄ in der Summe drei Kohlenstoffatome und ein quartäres Stickstoffatom, das den Rest R⁵ mit der Bedeutung einer C₁₋₄-Alkyl-, C₁₋₄-Hydroxyalkyl-, C₁₋₄-Carboxyalkyl-, C₁₋₄-Sulfoalkyl-, einer Aryl-, Aralkyl- oder Heteroarylgruppe oder auch eines N-Oxids trägt, und
   Y⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat, Tetrachlorzinkat oder Oxid eines N-Oxids bedeuten,
   und
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die quartemierten Azafluorenone der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die quartemierten Azafluorenone der Formel I und die Verbindungen der Komponente B können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiel

### Herstellung von 1-Methyl-5-oxo-indeno[1,2-b]pyridinium-trifluormethansulfonat

10,0 g (54,9 mmol) 1-Methyl-5-oxo-indeno[1,2-b]pyridin-4-aza-fluoren-9-on und 11,5 (70,5 mmol) Trifluormethansulfonsäuremethylester wurden in 50 ml Toluol gelöst und unter Rühren 16 Std. auf 80°C erwärmt, auf Raumtemperatur abgekühlt, mit warmem Toluol ausgewaschen und im Ölpumpenvakuum getrocknet. Es wurden 19,0 g (100% d. Th.) 1-Methyl-5-oxo-indeno[1,2-b]pyridinium-trifluormethansulfonat als gelbliche Kristalle vom Schmp. 143°C erhalten (1H-NMR-Spektrum).

### Herstellung einer Färbelösung

Es wurde je eine Aufschlämmung von 5 mmol der oben hergestellten Verbindung, ggf. 5 mmol eines Oxidationsfarbstoffvorproduktes der Komponente B und 5 mmol Natriumacetat und einen Tropfen einer 20-%igen Fettalkylethersulfat-Lösung in 50 ml Wasser bei 60°C hergestellt. Die Aufschlämmungen wurden nach Abkühlen auf 30°C miteinander vermischt und mit verdünnter NaOH oder Salzsäure auf einen pH-Wert von 6 eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| Ausfärbungen mit 1-Methyl-5-oxo-indeno[1,2b]pyridinium-trifluormethansulfonat | | |
|---|---|---|
| **Komponente B** | **Farbe** | **Intensität** |
| - | braungelb | ++ |
| 2,5-Diaminotoluol x H₂SO₄ | dunkelviolettbraun | +++ |
| 2,4,5,6-Tetraaminopyrimidin x H₂SO₄ | dunkelbraun | ++(+) |
| 1,8-Bis-(2,5-diaminophenoxy)-3,6-dioxaoctan x 4 HCl | dunkelgrünbraun | +++ |
| 2-Methylamino-3-amino-6-methoxypyridin x 2 HCl | braunschwarz | +++ |
| 2-(2,5-Diaminophenyl)-ethanol x H₂SO₄ | dunkelrotbraun | +++ |
| 2-Aminomethyl-4-aminophenol x 2 HCl | orangebraun | ++ |
| N,N-Bis-(2-hydroxyethyl)-p-phenylendiamin x H₂SO₄ | schwarz | +++ |
| 4,4'-Diaminodiphenylamin x H₂SO₄ | dunkelgrau | ++(+) |
| 3,5-Diamino-2,6-dimethoxypyridin x 2 HCl | schwarz | +++ |

## Patentansprüche

1. Mittel zum Färben von keratinhaltigen Fasern, insbesondere menschlichen Haaren, enthaltend als färbende Komponente mindestens ein quartemiertes Azafluorenon mit der Formel I, 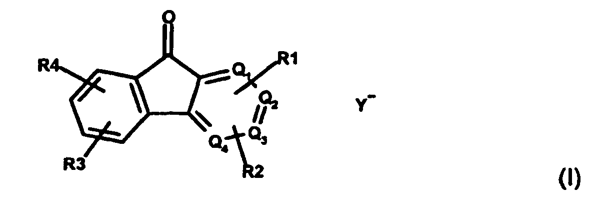 in der R¹, R², R³ und R⁴ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe wobei auch zwei der Reste gemeinsam einen ankondensierten aromatischen Ring bilden können,
Q₁, Q₂, Q₃ und Q₄ in der Summe drei Kohlenstoffatome und ein quartäres Stickstoffatom, das den Rest R⁵ mit der Bedeutung einer C₁₋₄-Alkyl-, C₁₋₄-Hydroxyalkyl-, C₁₋₄-Carboxyalkyl-, C₁₋₄-Sulfoalkyl-, einer Aryl-, Aralkyl- oder Heteroarylgruppe oder auch eines N-Oxids trägt, und
Y⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat, Tetrachlorzinkat oder Oxid eines N-Oxids bedeuten;
und
zusätzlich mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder mindestens eine CH-aktive Verbindung.

2. Mittel nach Anspruch 1, **dadurch gekennzeichnet, daß** als Verbindungen mit der Formel I Trifluormethansulfonate, Benzolsulfonate, p-Toluolsulfonate, Methansulfonate, Perchlorate, Sulfate, Chloride, Bromide, lodide, Tetrachlorzinkate, Hexafluorphosphate oder Tetrafluorborate von 1-Methyl-5-oxo-indeno[1,2-b]pyridinium, 4-methyl-4-azonio-9-fluorenon, 1-Ethyl-5-oxo-indeno[1,2-b]pyridinium, 1-Benzyl-5-oxoindeno[1,2-b]pyridinium, 2-Methyl-5-oxo-indeno[1,2-c]pyridinium, 2-Methyl-9-oxo-indeno[2,1-c]pyridinium oder 1-Methyl-9-oxo-ideno[2,1-b]pyridinium sowie deren beliebigen Gemische eingesetzt werden.

3. Mittel nach einem der Ansprüche 1 oder 2, **dadurch gekennzeichnet, daß** die quartemierten Azafluorenone mit der Formel I in einer Menge von 0,03 bis 65 mmol, insbesondere von 1 bis 40 mmol, bezogen auf 100 g des gesamten Färbemittels, enthalten sind.

4. Mittel nach Anspruch 1 bis 3, **dadurch gekennzeichnet, daß** die weitere Verbindung ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, - phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 2-Hydroxymethyl-4-aminophenol, 2-Diethylaminomethyl-4-aminophenol, 2-Dimethylaminomethyl-4-aminophenol, 2,6-Dichlor-4-aminophenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzolpentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyltetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethylpyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, - acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure, und
CH-aktiven Verbindungen ausgewählt aus der Gruppe, bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Ethyl-2-chinaldiniumiodid, 1,4-Dimethylchinolinium-iodid, 1-Methyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

5. Mittel nach Anspruch 1 bis 4, **dadurch gekennzeichnet, daß** die weitere Verbindung ausgewählt ist aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie jeweils aus den vorzugsweise mit anorganischen Säuren gebildeten physiologisch verträglichen Salzen dieser Verbindungen.

6. Mittel nach einem der Ansprüche 1 bis 5, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

7. Mittel nach einem der Ansprüche 1 bis 6, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

8. Mittel nach einem der Ansprüche 1 bis 7, **dadurch gekennzeichnet, daß** es Oxidationsmittel, insbesondere H₂O₂, in einer Menge von 0,01 bis 6 Gew.-%, bezogen auf die Anwendungslösung, enthält.

9. Mittel nach einem der Ansprüche 1 bis 8, **dadurch gekennzeichnet, daß** es anionische, zwitterionische oder nichtionische Tenside enthält.

10. Verwendung von mindestens einem quartemierten Azafluorenon mit der Formel I, 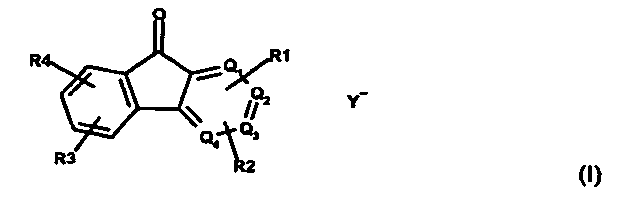 in der R¹, R², R³ und R⁴ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe, wobei auch zwei der Reste gemeinsam einen ankondensierten aromatischen Ring bilden können,
Q₁, Q₂, Q₃ und Q₄ in der Summe drei Kohlenstoffatome und ein quartäres Stickstoffatom, das den Rest R⁵ mit der Bedeutung einer C₁₋₄-Alkyl-, C₁₋₄-Hydroxyalkyl-, C₁₋₄-Carboxyalkyl-, C₁₋₄-Sulfoalkyl-, einer Aryl-, Aralkyl- oder Heteroarylgruppe oder auch eines N-Oxids trägt, und
Y⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat, Tetrachlorzinkat oder Oxid eines N-Oxids bedeuten,
als eine färbende Komponente zum Färben keratinhaltiger Fasern.

11. Verwendung nach Anspruch 10, **dadurch gekennzeichnet, daß** als Verbindungen mit der Formel I Trifluormethansulfonate, Benzolsulfonate, p-Toluolsulfonate, Methansulfonate, Perchlorate, Sulfate, Chloride, Bromide, Iodide, Tetrachlorzinkate, Hexafluorphosphate oder Tetrafluorborate von 1-Methyl-5-oxo-indeno[1,2-b]pyridinium, 4-methyl-4-azonio-9-fluorenon, 1-Ethyl-5-oxo-indeno[1,2-b]pyridinium, 1-Benzyl-5-oxo-indeno[1,2-b]pyridinium, 2-Methyl-5-oxo-indeno[1,2-c]pyridinium, 2-Methyl-9-oxo-indeno[2,1-c]pyridinium oder 1-Methyl-9-oxo-ideno[2,1-b]pyridinium sowie deren beliebigen Gemische eingesetzt werden.

12. Verwendung nach einem der Ansprüche 10 oder 11, **dadurch gekennzeichnet, daß** die quartemierten Azafluorenone mit der Formel I in einer Menge von 0,03 bis 65 mmol, insbesondere von 1 bis 40 mmol, bezogen auf 100 g des gesamten Färbemittels, enthalten sind.

13. Verwendung eines Mittels nach einem der Ansprüche 1 bis 9 zum Färben von keratinhaltigen Fasern, insbesondere menschlichen Haaren.

14. Verfahren zum Färben von keratinhaltigen Fasern, insbesondere menschlichen Haaren, worin ein Färbemittel, enthaltend
A) mindestens ein quartemiertes Azafluorenon mit der Formel I, 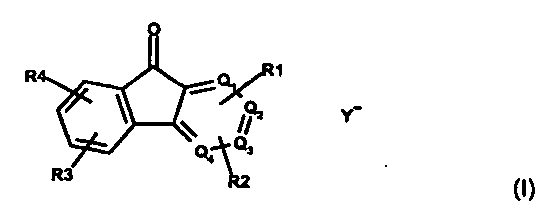 in der R¹, R², R³ und R⁴ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe, wobei auch zwei der Reste gemeinsam einen ankondensierten aromatischen Ring bilden können,
Q₁, Q₂, Q₃ und Q₄ in der Summe drei Kohlenstoffatome und ein quartäres Stickstoffatom, das den Rest R⁵ mit der Bedeutung einer C₁₋₄-Alkyl-, C₁₋₄-Hydroxyalkyl-, C₁₋₄-Carboxyalkyl-, C₁₋₄-Sulfoalkyl-, einer Aryl-, Aralkyl- oder Heteroarylgruppe oder auch eines N-Oxids trägt, und
Y⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat, Tetrachlorzinkat oder Oxid eines N-Oxids bedeuten,
und
B) mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Preparation for colouring keratin-containing fibres, more particularly human hair, containing as colouring component a quaternized azafluorenone corresponding to formula I: 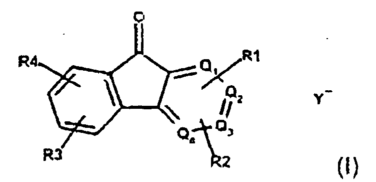 in which R¹, R², R³ and R⁴ represent a hydrogen atom, a halogen atom, a C₁₋₄ alkyl, C₁₋₄ alkoxy or C₁₋₄ hydroxyalkyoxy group, a hydroxy, nitro, amino group which may be substituted by C₁₋₄ alkyl groups, or a C₁₋₄ acyl group; two of the substituents together may even form a fused aromatic ring,
Q₁, Q₂. Q₃ and Q₄ contain a total of three carbon atoms and one quaternary nitrogen atom which bears the substituent R⁵ with the meaning of a C₁₋₄ alkyl, C₁₋₄ hydroxyalkyl, C₁₋₄ carboxyalkyl, C₁₋₄ sulfoalkyl. aryl, aralkyl or heteroaryl group or even an N oxide, and
Y⁻ is halide, C₁₋₄ alkyl sulfate. arene sulfate, C₁₋₄ alkanesulfonate, C₁₋₄ perfluoroalkane sulfonate, perhalogenate, sulfate, hydrogen sulfate, tetrafluoroborate, hexafluorophosphate, tetrachlorozincate or oxide of an N oxide;
and
additionally at least one compound containing a primary or secondary amino group or hydroxy group selected from primary or secondary aromatic amines, nitrogen-containing heterocyclic compounds and aromatic hydroxy compounds and/or at least one CH-active compound.

2. Preparation as claimed in claim 1, **characterized in that** trifluoromethaneulfonates, benzenesulfonates, p-toluenesulfonates, methanesulfonates, perchlorates, sulfates, chlorides. bromides, iodides, tetrachlorozincates, hexafluorophosphates or tetrafluoroborates of 1-methyl-5-oxo-indeno[1,2-b]pyridinium-(4-methyl-4-azonio-9-fluorenone), 1-ethyl-5-oxo-indeno[1,2-b]pyridinium, 1-benzyl-5-oxo-indeno[1,2-b]-pyridinium, 2-methyl-5-oxo-indeno[1,2-c]pyridinium, 2-methyl-9-oxo-indeno[2,1-c]pyridinium or 1-methyl-9-oxo-ideno[2,1-b]pyridinium and mixtures thereof are used as the compounds of formula I.

3. Preparation as claimed in claim 1 or 2, **characterized in that** the quaternized azafluorenones corresponding to formula I are present in a quantity of 0.03 to 65 mmol and more particularly in a quantity of 1 to 40 mmol, based on 100 g of the colorant as a whole.

4. Preparation as claimed in claims 1 to 3, **characterized in that** the other compound is selected from primary or secondary amines from the group consisting of N-(2-hydroxyethyl)-N-ethyl-, N-(2-methoxyethyl-), 2,3-, 2,4-, 2,5-dichloro-p-phenylenediamine, 2-chloro-p-phenylenediamine, N,N-bis-(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)-ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 5-(2-hydroxyethylamino)-4-methoxy-2-methyl-, 4-amino-2-aminomethyl phenol, 2-hydroxymethyl-4-aminophenol, 2-diethylaminomethyl-4-aminophenol, 2-dimethylaminomethyl-4-aminophenol, 2,6-dichloro-4-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulfonic acid, 3-amino-4-hydroxybenzenesulfonic acid, 4-amino-3-hydroxynaphthalene sulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulfate, 4,6-diaminopyrogallol dihydrochloride, 3,5-diamino-4-hydroxypyrocatechol sulfate, aromatic nitriles, anilines, moe particularly anilines containing nitro groups, such as 4-nitroaniline, 4-nitro-1,3-phenylenediamine, 2-nitro-4-amino-1-(2-hydroxyethylamino)-benzene, 2-nitro-1-amino-4-[bis-(2-hydroxyethyl)-amino]-benzene, 4-amino-2-nitrodiphenylamine-2'-carboxylic acid, 2-amino-6-chloro-4-nitrophenol, 1-amino-5-chloro-4-(2-hydroxyethylamino)-2-nikrobenzene, aromatic anilines and phenols containing another aromatic group, such as 4,4'-diaminostilbene dihydrochloride, 4,4'-diaminostilbene-2,2'-disulfonic acid, Na salt, 4,4'-diaminodiphenylmethane, -sulfide, -sulfoxide, -amine, 4,4'-diaminodiphenylamine-2-sulfonic acid, 4,4'-diaminobenzophenone, -diphenylether, 3,3',4,4'-tetraaminodiphenyl tetrahydrochloride, 3,3',4,4'-tetraaminobenzophenone, 1,3-bis-(2,4-diaminophenoxy)-propane tetrahydrochloride, 1,8-bis-(2,5-diaminophenoxy)-3,6-dioxaoctane tetrahydrochloride, 1,3-bis-(4-aminophenylamino)-propane, -2-propanol, 1,3-bis-[N-(4-aminophenyl)-2-hydroxyethylamino]-2-propanol, N,N-bis-[2-(4-aminophenoxy)-ethyl]methylamine trihydrochloride,
nitrogen-containing heterocyclic compounds selected from the group consisting of 2-, 3-, 4-amino-, 2-amino-3-hydroxy-, 2,6-diamino-, 2,5-diamino-, 2.3-diamino-, 2-dimethylamino-5-amino-, 3-amino-2-methylamino-6-methoxy-. 2,3-diamino-6-methoxy-, 3,5-diamino-2,6-dimethoxy-, 2,4,5-triamino-, 2,6-dihydroxy-3,4-dimethylpyridin, 4,5,6-triamino-, 2-hydroxy-4,5,6-triamino-, 4-hydroxy-2,5,6-triamino-, 2,4,5,6-tetraamino-, 2-methylamino-4.5,6-triamino-. 2,4-, 4,5-diamino-, 2-amino-4-methoxy-6-methyl pyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 2-,3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2.5-dihydroxy-4-morpholinoaniline and indole and indoline derivatives, such as 4-, 5-, 6-, 7-aminoindole, 4-. 5-, 6-. 7-hydroxyindole, 5,6-dihydroxyindole, 5,6-dihydroxyindoline and 4-hydroxyindoline, and from the physiologically compatible salts of these compounds formed with preferably inorganic acids,
aromatic hydroxy compounds selected from the group consisting of 2-, 4-, 5-methylresorcinol, 2,5-dimethylresorcinol, resorcinol, 3-methoxyphenol, pyrocatechol, hydroquinone, pyrogallol, phloroglucinol,hHydroxyhydroquinone, 2-, 3-, 4-methoxy-, 3-dimethylamino-, 2-(2-hydroxyethyl)-, 3,4-methylenedioxyphenol. 2,4-, 3,4-dihydroxybenzoic acid, -phenylacetic acid. gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulfonic acid, 3.6-dihydroxy-2,7-naphthalenesulfonic acid, and
CH-active compounds selected from the group consisting of 1,2,3,3-tetramethyl-3H-indolium iodide, 1,2,3.3-tetramethyl-3H-indolium-p-toluenesulfonate, 1,2,3,3-tetramethyl-3H-indolium methanesulfonate, Fischer's base (1,3,3-trimethyl-2-methyleneindoline), 2,3-dimethyl-benzothiazolium iodide, 2,3-dimethyl benzothiazolium-p-toluenesulfonate. rhodanine, rhodanine-3-acetic acid, 1-ethyl-2-quinaldinium iodide, 1,4-dimethylquinolinium iodide, 1-methyl-2-quinaldinium iodide, 1.4-dimethylquinolinium iodide, barbituric acid, thiobarbituric acid, 1,3-dimethylthiobarbituric acid, 1,3-diethylthiobarbituric acid, diethylthiobarbituric acid, oxindole, 3-indoxylacetate, coumaranone and 1-methyl-3-phenyl-2-pyrazolinone.

5. Preparation as claimed in claims 1 to 4, **characterized in that** the other compound is selected from the group consisting of N-(2-hydroxyethyl)-N-ethyl-, 2-chloro-p-phenylenediamine, N,N-bis-(2-hydroxyethyl)-p-phenylenediamine, 4-aminophenol, p-phenylenediamine, 2-(2,5-diaminophenyl)-ethanol, 2,5-diaminotoluene, 3,4-methylenedioxyaniline, 2-amino-4-(2-hydroxyethylamino)-anisole, 2-(2,4-diaminophenoxy)-ethanol; 3-amino-2,4-dichloro-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 5,6-dihydroxyindoel and 5,6-dihydroxyindoline and from the physiologically compatible salts of these compounds formed preferably with inorganic acids

6. Preparation as claimed in any of claims 1 to 5, **characterized in that** it contains substantive dyes from the group of nitrophenylenediamines, nitroaminophenols, anthraquinones or indophenols in a quantity of preferably 0.01 to 20% by weight. based on the colorant as a whole.

7. Preparation as claimed in any of claims 1 to 6, **characterized in that** ammonium or metal salts selected from the group of formates, carbonates, halides, sulfates, butyrates, valerates, caproates, acetates, lactates, glycolates, tartrals, citrates, gluconates, propionates, phosphates and phosphonates of alkali metals, such as potassium, sodium or Ithium, alkalie earth metals, such as magnesium, calcium, strontium or barium, or of aluminium, manganese, iron, cobalt, copper or zinc are added.

8. Preparation as claimed in any of claims 1 to 7, **characterized in that** it contains oxidizing agents, more particularly H₂O₂, in a quantity of 0.01 to 6% by weight, based on the solution applied.

9. Preparation as claimed in any of claims 1 to 8, **characterized in that** it contains anionic, zwitterionic or nonionic surfactants.

10. The use of at least one quaternized azafluorenone corresponding to formula I: 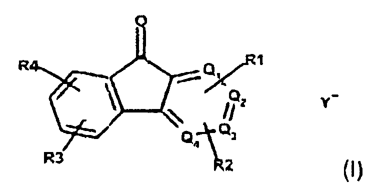 in which R¹, R², R³ and R⁴ represent a hydrogen atom, a halogen atom, a C₁₋₄ alkyl, C₁₋₄ alkoxy or C₁₋₄ hydroxyalkyoxy group, a hydroxy, nitro, amino group which may be substituted by C₁₋₄ alkyl groups, or a C₁₋₄ acyl group: two of the substituents together may even form a fused aromatic ring.
Q₁, Q₂, Q₃ and Q₄ contain a total of three carbon atoms and one quaternary nitrogen atom which bears the substituent R⁵ with the meaning of a C₁₋₄ alkyl, C₁₋₄ hydroxyalkyl, C₁₋₄ carboxyalkyl, C₁₋₄ sulfoalkyl, aryl, aralkyl or heteroaryl group or even an N oxide, and
Y⁻ is halide, C₁₋₄ alkyl sulfate, arene sulfate, C₁₋₄ alkanesulfonate, C₁₋₄ perfluoroalkane sulfonate, perhalogenate, sulfate, hydrogen sulfate, tetrafluoroborate, hexafluorophosphate, tetrachlorozincate or oxide of an N oxide,
as a colouring component for colouring keratin-containing fibres.

11. The use claimed in claim 10, **characterized in that** trifluoromethaneulfonates, benzenesulfonates, p-toluenesulfonates, methanesulfonates, perchlorates, sulfates, chlorides, bromides, iodides, tetrachlorozincates, hexafluorophosphates or tetrafluoroborates of 1-methyl-5-oxo-indeno[1,2-b]pyridinium-(4-methyl-4-azonio-9-fluorenone). 1-ethyl-5-oxo-indeno[1,2-b]pyridinium, 1-benzyl-5-oxo-indeno[1,2-b]-pyridinium. 2-methyl-5-oxo-indeno[1,2-c]pyridinium, 2-methyl-9-oxo-indeno[2,1-c]pyridinium or 1-methyl-9-oxo-ideno[2.1-b]pyridinium and mixtures thereof are used as the compounds of formula I.

12. The use claimed in claim 10 or 11, **characterized in that** the quaternized azafluorenones corresponding to formula I are present in a quantity of 0.03 to 65 mmol and more particularly in a quantity of 1 to 40 mmol, based on 100 g of the colorant as a whole.

13. The use of the preparation claimed in any of claims 1 to 9 for colouring keralin-containing fibres, more particularly human hair.

14. A process for colouring keratin-containing fibres, more particularly human hair, in which a colorant containing
A) al. least one quaternized azafluorenone corresponding to formula I: 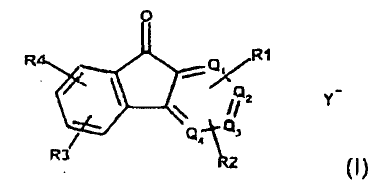 in which R¹, R², R³ and R⁴ represent a hydrogen atom, a halogen atom, a C₁₋₄ alkyl, C₁₋₄ alkoxy or C₁₋₄ hydroxyalkyoxy group, a hydroxy, nitro, amino group which may be substituted by C₁₋₄ alkyl groups, or a C₁₋₄ acyl group: two of the substituents together may even form a fused aromatic ring,
Q₁, Q₂, Q₃ and Q₄ contain a total of three carbon atoms and one quaternary nitrogen atom which bears the substituent R⁵ with the meaning of a C₁₋₄ alkyl, C₁₋₄ hydroxyalkyl, C₁₋₄ carboxyalkyl, C₁₋₄ sulfoalkyl, aryl, aralkyl or heteroaryl group or even an N oxide, and
Y⁻ is halide, C₁₋₄ alkyl sulfate, arene sulfate, C₁₋₄ alkanesulfonate, C₁₋₄ perfluoroalkane sulfonate, perhalogenate, sulfate, hydrogen sulfate, tetrafluoroborate, hexafluorophosphate, tetrachlorozincate or oxide of an N oxide;
and
B) at least one compound containing a primary or secondary amino group or hydroxy group selected from primary or secondary aromatic amines, nitrogen-containing heterocyclic compounds and aromatic hydroxy compounds and/or at least one CH-active compound
and typical cosmetic ingredients is applied to the keratin-containing fibres, left thereon for a certain time, typically ca. 30 minutes, and then rinsed oul or washed out again with a shampoo.

## Revendications

1. Agent pour la teinture de fibres kératiniques, en particulier de cheveux humains contenant, à titre de composant de teinture, au moins une azafluorénone quaternisée répondant à la formule I 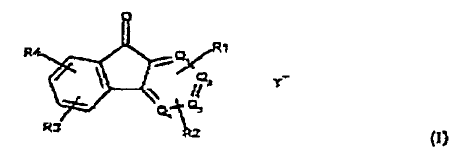 dans laquelle R¹, R², R³ et R⁴ représentent un atome d'hydrogène, un atome d'halogène, un groupe alkyle en C₁-C₄, un groupe alcoxy en C₁-C₄ ou un groupe hydroxyalcoxy en C₁-C₄, un groupe hydroxyle, un groupe nitro, un groupe amino qui peut être substitué par des groupes alkyle en C₁-C₄, ou un groupe acyle en C₁-C₄, deux des radicaux pouvant également former ensemble un noyau aromatique condensé,
Q₁, Q₂, Q₃ et Q₄ représentent au total trois atomes de carbone et un atome d'azote quaternaire qui porte le radical R⁵ qui représente un groupe alkyle en C₁-C₄, un groupe hydroxyalkyle en C₁-C₂, un groupe carboxyalkyle en C₁-C₄, un groupe sulfoalkyle en C₁-C₄ un groupe aryle, un groupe aralkyle ou un groupe hétéroaryle ou encore un N-oxyde, et
Y⁻ représente un halogénure, un alkyl(en C₁-C₄)sulfate, un arénesulfate, un alcane(en C₁-C₄)sulfonate, un perfluoroalcane(en C₁-C₄)sulfonate, un perhalogénate, un sulfate, un hydrogénosuffate, un tétrafluoroborate, un hexafluorophosphate, un tétrachloro-zincate ou un oxyde d'un N-oxyde ;
et
en outre au moins un composé comprenant un groupe amino primaire ou secondaire ou un groupe hydroxyle, choisi parmi le groupe comprenant des amines aromatiques primaires ou secondaires, des composés hétérocycliques azotés et des composés hydroxylés aromatiques, et/ou au moins un composé CH-actif.

2. Agent selon la revendication 1, **caractérisé en ce qu'**on met en oeuvre, à titre de composés répondant à la formule I, des trifluorométhane-sulfonates, des benzénesulfonates, des p-toluènesulfonates, des méthanesulfonates, des perchlorates, des sulfates, des chlorures, des bromures, des iodures, des tétrachlorozincates, des hexafluorophosphates ou des tétrafluoroborates de 1-méthyl-5-oxo-indéno[1,2-b]-pyridinium, de 4-méthyl-4-azonio-9-fluorénone, de 1-éthyl-5-oxo-indéno[1,2-b]-pyridinium, de 1-benzyl-5-oxo-indéno[1,2-b]-pyridinium, of 2-méthyl-5-oxo-indéno[1,2-c]-pyridinium, de 2-méthyl-9-oxo-indéno[2,1 c]-pyridinium ou de 1-méthyl-9-oxo-indéno[2,1-b]-pyridinium, ainsi que l'un quelconque de leurs mélanges.

3. Agent selon l'une quelconque des revendications 1 ou 2, **caractérisé en ce que** les azafluorénones quaternisées répondant à la formule I sont contenues en une quantité de 0,03 à 65 millimoles, en particulier de 1 à 40 millimoles, rapportés à 100 g de la totalité de la teinture.

4. Agent selon les revendications 1 à 3, **caractérisé en ce que** le composé supplémentaire est choisi parmi le groupe comprenant des amines primaires ou secondaires choisies parmi le groupe constitué par la N-(2-hydroxyéthyl)-N-éthyl-, la N-(2-méthoxyéthyl)-, la 2,3-, la 2,4-, la 2,5-dichloro-p-phénylènediamine, la 2-chloro-p-phénylènediamine, la N,N-bis-(2-hydroxyéthyl)-p-phénylènediamine, le dibromhydrate de la 2,5-dihydroxy-4-morpholino-aniline, le 2-, le 3-, le 4-aminophénol, la o-, la m-, la p-phénylènediamine, le 2,4-diaminophénoxyéthanol, le 2-(2,5-diaminophényl)-éthanol, le 2,5-diamino-toluène, -phénol, -phénéthol, la 4-méthylamino-, la 3-amino-4-(2'-hydroxyéthyloxy), la 3,4-mèthylénediamino-, la 3,4-méthylène-dioxyaniline, le 3-amino-2,4-dichloro-, le 4-méthylamino-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 5-(2-hydroxyéthylamino)-4-méthoxy-2-méthyl-, le 4-amino-2-aminométhyl-phénol, le 2-hydroxyméthyl-4-arninophénol, le 2-diéthylaminométhyl-4-aminophénol, le 2-diméthylaminométhyl-4-aminophénol, le 2,6-dichloro-4-aminophénol, le 1,3-diamino-2,4-diméthoxybenzène, l'acide 2-, l'acide 3-, l'acide 4-arninobenzoïque, l'acide 2-, l'acide 3-, l'acide 4-aminophénylacétique, l'acide 2,3-, l'acide 2,4-, l'acide 2,5-, l'acide 3,4-, l'acide 3,5-diaminobenzoïque, l'acide 4-, l'acide 5-aminosalicylique, l'acide 3-amino-4-hydroxy-, l'acide 4-amino-3-hydroxy-benzoïque, l'acide 2-, l'acide 3-, l'acide 4-aminobenzènesulfonique, l'acide 3-amino-4-hydroxybenzènesulfonique, l'acide 4-amino-3-hydroxy-naphtalène-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-amino-isophtalique, le 1,3,5-, le 1,2,4-triaminobenzène, le tétrachlorhydrate du 1,2,4,5-tétraaminobenzène, le trichlorhydrate du 2,4,5-triaminophénol, le pentachlorhydrate du pentaaminobenzène, l'hexachlorhydrate de l'hexaaminobenzène, le trichlorhydrate du 2 4,6-triaminorésorcinol, le sulfate du 4,5-diaminopyrocatéchol, le dichlorhydrate du 4,6-diaminopyrogallol, le sulfate du 3,5-diamino-4-hydroxypyrocatéchol, des nitriles aromatiques, des anilines, en particulier des anilines contenant un ou plusieurs groupes nitro, telles que la 4-nitroaniline, la 4-nitro-1,3-phénylènediamine, le 2-nitro-4-amino-1-(2-hydraxyéthylamino)-benzène, le 2-nitro-1-amino-4-[bis-(2-hydroxyéthyl)-aminol-benzène, l'acide 4-amino-2-nitrodiphénylamine-2'-carboxylique, le 2-amino-6-chloro-4-nitrophénol, le 1-amino-5-chloro-4-(2-hydroxyéthylamino)-2-nitrobenzène, des anilines aromatiques, respectivement des phénols aromatiques possédant un radical aromatique supplémentaire, tels que le dichlorhydrate du 4,4'-diaminostilbène, l'acide 4,4'-diaminostilbéne-2,2'-disulfonique, sel de sodium, le (la) 4,4'-diaminodiphényl-méthane, -sulfure, -sulfoxyde, -amine, l'acide 4,4'-diaminodiphénylamine-2-sulfonique, la 4,4'-diaminobenzophénone, l'éther 4,4'-diaminodiphénylique, le tétrachlorhydrate du 3,3',4,4'-tétraaminodiphényle, la 3,3',4,4'-tétraaminobenzophénone, le tétrachlorhydrate du 1,3-bis-(2,4-diaminophénoxy)-propane, le tétrachlorhydrate du 1,8-bis-(2,5-diaminophénoxy)-3,6-dioxa-octane, le 1,3-bis-(4-aminophénylamino)-propane, -2-propanol, le 1,3-bis-[N-(4-aminophényl)-2-hydroxyéthylamino]-2-propanol, le trichlorhydrate de la N,N-bis-[2-(4-amino phénoxy)-éthyl]méthylamine,
des composés hétérocycliques azotés choisis parmi le groupe constitué par la 2-, la 3-, la 4-amino-, la 2-amino-3-hydroxy-, la 2,6-diamino-, la 2,5-diamino-, la 2,3-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,4,5-triamino-, la 2,6-dihydroxy-3,4-diméthyl-pyridine, la 4,5,6-triamino-, la 2-hydroxy-4,5,6-triamino-, la 4-hydroxy-2,5,6-triamino-, la 2,4,5,6-tétraamino-, la 2-méthylamino-4,5,6-triamino-, la 2,4-, la 4,5-diamino-, la 2-amino-4-méthoxy-6-méthyl-pyrimidine, le 2.3,4-triméthylpyrrole, le 2,4-diméthyl-3-éthyl-pyrrole, le 3,5-diaminopyrazole, -1,2,4-triazole, le 3-amino-, le 3-amino-5-hydroxypyrazole, la 2-, la 3-, la 8-aminoquinoléine, la 4-amino-quinaldine, l'acide 2-, l'acide 6-aminonicotinique, la 5-amino-isoquinoléine, le 5-, le 6-amino-indazole, le 5-, le 7-amino-benzimidazole, -benzothiazole, la 2,5-dihydroxy-4-morpholino-aniline, ainsi que des dérivés d'indole et d'indoline, tels que le 4-, le 5-, le 6-, le 7-amino-indole, le 4-, le 5-, 6-, le 7-hydroxyindole, le 5,6-dihydroxyindole, la 5,6-dihydroxyindoline et la 4-hydroxyindoline, ainsi que, respectivement, par les sels physiologiquement acceptables de ces composés, formés avec de préférence des acides inorganiques,
des composés hydroxylés aromatiques choisis parmi le groupe constitué par le 2-, le 4-, le 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-, le 3-, le 4-méthoxy-, le 3-diméthylamino-, le 2-(2-hydroxyéthyl)-, le 3,4-méthylènedioxyphénol, l'acide 2,4-, l'acide 3,4-dihydroxybenzoïque, -phénylacétique, l'acide gallique, l'acide 2,4,6-trihydroxybenzoïque, la 2,4,6-trihydroxyacétophénone, le 2-, le 4-chlororésorcinol, le 1-naphtol, le 1,5-, le 2,3-, le 2,7-dihydroxynaphtalène, l'acide 6-diméthylamino-4-hydroxy-2-naphtalènesulfonique, l'acide 3,6-dihydroxy-2,7-naphtalènesulfonique, et
des composés CH-actifs choisis parmi le groupe constitué par l'iodure du 1,2,3,3-tétraméthyl-3H-indolium, le p-toluènesulfonate du 1,2,3,3-tétraméthyl-3H-indolium, le méthanesulfonate du 1,2,3,3-tétraméthyl-3H-indolium, la base de Fischer (1,3,3-triméthyl-2-méthylène-indoline), l'iodure du 2,3-diméthyl-benzothiazolium, le p-toluènesulfonate du 2,3-diméthyl-benzothiazolium, la rhodanine, l'acide rhodanine-3-acétique, l'iodure du 1-éthyl-2-quinaldinium, l'iodure du 1,4-diméthylquinolinium, l'iodure du 1-méthyl-2-quinaldinium, l'iodure du 1,4-diméthylquinolinium, l'acide barbiturique, l'acide thiobarbiturique, l'acide 1,3-diméthylthiobarbiturique, l'acide 1,3-diéthylthiobarbiturique, l'acide diéthylthiobarbiturique, l'oxoindole, le 3-indoxylacétate, la coumaranone et la 1-méthyl-3-phényl-2-pyrazolinone,

5. Agent selon les revendications 1 à 4, **caractérisé en ce que** le composé supplémentaire est choisi parmi le groupe constitué par la N-(2-hydroxyéthyl)-N-éthyl-, la 2-chloro-p-phénylène-diamine, la N,N-bis-(2-hydroxyéthyl)-p-phénylènediamine, le 4-aminophénol, la p-phénylénediamine, le 2-(2,5-diamino-phényl)-éthanol, le 2,5-diaminotoluène, la 3,4-méthylènedioxyaniline, le 2-amino-4-(2-hydroxyéthylamino)-anisol, le 2-(2,4-diaminophénoxy)-éthanol, le 3-amino-2,4-dichloro-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 2-aminométhyl-4-aminophénol, le 2-diéthyl-4-aminophénol, le 2-diméthylaminométhyl-4-aminophénol, Ile 2,6-dichloro-4-aminophénol, le 2-hydroxyméthyl-4-aminophénol, le 3,4-méthylènedioxyphénol, l'acide 3,4-diaminobenzoïque, la 2,5-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,6-dihydroxy-3,4-diméthylpyridine, la 2-hydroxy-4,5,6-triamino-, la 4-hydroxy-2,5,6-triamino-, la 2,4,5,6-tétraamino-, la 2-méthylamino-4,5,6-triaminopyrimidine, le 3,5-diamino-pyrazole, le 3-amino-5-hydroxypyrazole, le 5,6-dihydroxyindole et la 5,6-dihydroxyindoline, ainsi que, respectivement, par les sels physiologiquement acceptables de ces composés, formés avec de préférence des acides inorganiques.

6. Agent selon l'une quelconque des revendications 1 à 5, **caractérisé en ce qu'**il contient des colorants montant directement sur la fibre, choisis parmi le groupe comprenant des nitrophénylènediamines, des nitroaminophénols, des anthraquinones ou des indophénols, de préférence en une quantité de 0,01 à 20 % en poids, rapportés à la totalité de la teinture.

7. Agent selon l'une quelconque des revendications 1 à 6, **caractérisé en ce qu'**on ajoute des sels d'ammonium ou des sels métalliques choisis parmi le groupe des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum, ou encore de l'aluminium, du manganèse, du fer, du cobalt, du cuivre ou du zinc.

8. Agent selon l'une quelconque des revendications 1 à 7, **caractérisé en ce qu'**il contient un ou plusieurs agents d'oxydation, en particulier du H₂O₂, en une quantité de 0,01 à 6 % en poids, rapportés à la solution d'utilisation.

9. Agent selon l'une quelconque des revendications 1 à 8, **caractérisé en ce qu'**il contient des agents tensioactifs anioniques, amphotères ou non ioniques.

10. Utilisation d'au moins une azafluorénone quaternisée répondant à la formule I 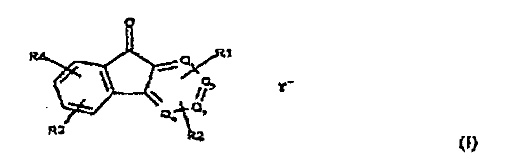 dans laquelle R¹, R², R³ et R⁴ représentent un atome d'hydrogène, un atome d'halogène, un groupe alkyle en C₁-C₄, un groupe alcoxy en C₁-C₄ ou un groupe hydroxyalcoxy en C₁-C₄, un groupe hydroxyle, un groupe nitro, un groupe amino qui peut être substitué par des groupes alkyle en C₁-C₄, ou un groupe acyle en C₁-C₄, deux des radicaux pouvant également former ensemble un noyau aromatique condensé,
Q₁, Q₂, Q₃ et Q₄ représentent au total trois atomes de carbone et un atome d'azote quaternaire qui porte le radical R⁵ qui représente un groupe alkyle en C₁-C₄, un groupe hydroxyalkyle en C₁-C₄, un groupe carboxyalkyle en C₁-C₄, un groupe sulfoalkyle en C₁-C₄ un groupe aryle, un groupe aralkyle ou un groupe hétéroaryle ou encore un N-oxyde, et
Y' représente un halogénure, un alkyl(en C₁-C₄)sulfate, un arène-sulfate, un alcane(en C₁-C₄)sulfonate, un perfluoroalcane(en Ci-C₄)sulfonate, un perhalogénate, un sulfate, un hydrogénosulfate, un tétrafluoroborate, un hexafluorophosphate, un tétrachloro-zincate ou un oxyde d'un N-oxyde ;
à titre de composant de teinture pour la teinture de fibres kératiniques.

11. Utilisation selon la revendication 10, **caractérisée en ce qu'**on met en oeuvre, à titre de composés répondant à la formule I, des trifluorométhane-sulfonates, des benzènesulfonates, des p-toluènesulfonates, des méthanesulfonates, des perchlorates, des sulfates, des chlorures, des bromures, des iodures, des tétrachlorozincates, des hexafluoro-phosphates ou des tétrafluoroborates de 1-méthyl-5-oxo-indéno[1,2-b]-pyridinium, de 4-méthyl-4-azonio-9-fluorénone, de 1-éthyl-5-oxo-indéno[1,2-b]-pyridinium, de 1-benzyl-5-oxo-indéno[1,2-b]-pyridinium, de 2-méthyl-5-oxo-indéno[1,2-c]-pyridinium, de 2-méthyl-9-oxo-indéno[2,1-c]-pyridinium ou de 1-méthyl-9-oxo-indéno[2,1-b]-pyridinium, ainsi que l'un quelconque de leurs mélanges.

12. Utilisation selon l'une quelconque des revendications 10 ou 11, **caractérisée en ce que** les azafluorénones quaternisées répondant à la formule I sont contenues en une quantité de 0,03 à 65 millimoles, en particulier de 1 à 40 millimoles, rapportés à 100 g de la totalité de la teinture.

13. Utilisation selon l'une quelconque des revendications 1 à 9, pour la teinture de fibres kératiniques, en particulier de cheveux humains.

14. Procédé pour la teinture de fibres kératiniques, en particulier de cheveux humains, dans lequel on applique sur les fibres kératiniques une teinture contenant
A) au moins une azafluorénone quaternisée répondant à la formule I 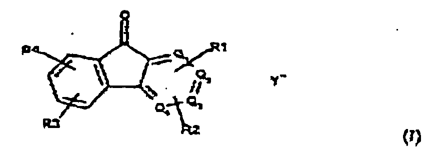 dans laquelle R¹, R², R³ et R⁴ représentent un atome d'hydrogène, un atome d'halogène, un groupe alkyle en C₁-C₄, un groupe alcoxy en C₁-C₄ ou un groupe hydroxyalcoxy en C₁-C₄, un groupe hydroxyle, un groupe nitro, un groupe amino qui peut être substitué par des groupes alkyle en C₁-C₄, ou un groupe acyle en C₁-C₄, deux des radicaux pouvant également former ensemble un noyau aromatique condensé,
Q₁, Q₂, Q₃ et Q₄ représentent au total trois atomes de carbone et un atome d'azote quaternaire qui porte le radical R⁵ qui représente un groupe alkyle en C₁-C₄, un groupe hydroxyalkyle en C₁-C₄, un groupe carboxyalkyle en C₁-C₄, un groupe sulfoalkyle en C₁-C₄ un groupe aryle, un groupe aralkyle ou un groupe hétéroaryle ou encore un N-oxyde, et
Y⁻ représente un halogénure, un alkyl(en C₁-C₄)sulfate, un arène-sulfate, un alcane(en C₁-C₄)sulfonate, un perfluoroalcane(en C₁-C₄)sulfonate, un perhalogénate, un sulfate, un hydrogénosulfate, un tétrafluoroborate, un hexafluorophosphate, un tétrachloro-zincate ou un oxyde d'un N-oxyde ;
et
B) au moins un composé comprenant un groupe amino primaire ou secondaire ou un groupe hydroxyle, choisi parmi le groupe comprenant des amines aromatiques primaires ou secondaires, des composés hétérocycliques azotés et des composés hydroxylés aromatiques, et/ou au moins un composé CH-actif,
ainsi que des constituants cosmétiques habituels, on l'applique sur les fibres kératiniques, on les laisse sur les fibres, habituellement pendant un laps de temps d'environ 30 minutes avant de procéder à un nouveau rinçage et à un lavage en profondeur avec un shampooing.
